# EUROPEAN PATENT APPLICATION

(11) **EP 1 978 088 A2**
(43) Date of publication of application: **08.10.2008**
(21) Application number: 07120892.0
(22) Date of filing: 16.11.2007
(51) Int. Cl.: C12M 1/34

(54) **Method and device for identifying a process wherein gaseous substances are released or consumed**

(30) Priority: 02.04.2007 EP 07105509
(71) Applicant: Consultatie Implementatie Technisch Beheer B.V., 7201 JB Zutphen (NL)
(72) Inventor: The designation of the inventor has not yet been filed
(74) Representative: Hatzmann, Martin

(57) **Abstract**

A device is provided for identifying a process wherein gaseous substances are exchanged with an atmosphere (6). The device comprises
- a sensor (10) for measuring as a function of time at least one parameter that is representative for a composition of said atmosphere,
- a signal processing facility (11) for
- identifying at least one portion in said function having relatively strong variations in comparison to neighboring portions, said at least one portion corresponding to a change in the process,
- identifying the process on the basis of the identification of said at least one portion.

## Description

### BACKGROUND OF THE INVENTION

### Field of the invention

The invention relates to a method for identifying a process wherein gaseous substances exchange with an atmosphere.

The invention further relates to a device for identifying a process wherein gaseous substances are released or consumed.

### Related Art

In the art, in order to detect a micro-organism such as a (pathogenic) bacteria or fungus, first a culture is provided, for example by fermentation. This can be done directly from an inoculation, which is delivering a sample through a septum in a vial by means of a syringe. It is also common practice to first derive pure strains by growing on an agar plate and using a pure colony as the inocculum for the fermentation.

Analysis can be performed visually when pure colonies are used as derived from an agar plate, or using some kind of measurement method at the end of a fermentation process. In the latter case, mostly culture broths that benefit specific bacteria strains are used in combination with a growing-mass method such as evolution of CO2 or assimilation of 02. The CO2 concentration can be measured optically or via pressure. In addition, it is common practice to first obtain pure colonies on an agar plate and then use the pure colonies to inoculate a set of fermentation broths in order to further determine the precise strain of bacteria.
Such a method is known for example from WO2006/079846. According to the method described therein a test sample is taken that is suspected of containing the bacteria.
Volatile bacterial products are collected from the test sample and subjected to gas chromatography. Therewith a gas chromatography system employing a surface acoustic wave detector is used.
A chromatographic profile is obtained for the test sample and compared to chromatographic profiles stored in a library to identify the bacteria.

This process takes a long time, which is often not available. Often, a risk of multiple infections is present of bacteria and fungi that are hard to discriminate. It would be desirable to have a rapid test available that would deliver initial results very soon.

A rapid test would also be desirable for other processes where gaseous substances are released or consumed, such as chemical processes. Such a test could indicate if the chemical process properly develops.

### SUMMARY OF THE INVENTION

It is an object of the invention to provide an improved method for identifying a process wherein gaseous substances are exchanged with an atmosphere.

It is a further object of the invention to provide an improved device for identifying a process wherein gaseous substances are exchanged with an atmosphere.

According to an aspect a method is provided for identifying a process wherein gaseous substances are exchanged with an atmosphere comprising the steps of
- measuring at least one parameter over time indicative for a composition of said atmosphere,
- measuring as a function of time at least one parameter indicative for a composition of said atmosphere,
- identifying at least one portion in said function having relatively strong variations in comparison to neighboring portions,
- identifying the process on the basis of the identification of said at least one portion.

According to a further aspect a device is provided for identifying a process wherein gaseous substances are exchanged with an atmosphere, comprising
- a sensor for measuring as a function of time at least one parameter that is representative for a composition of said atmosphere,
- a signal processing facility for
- identifying at least one portion in said function having relatively strong variations in comparison to neighboring portions,
- identifying the process on the basis of the identification of said at least one portion.

It is not necessary to know how the observed parameter depends on the composition of the atmosphere, as long as the dependency is reproducible, and a clear difference can be observed for the different processes that should be discriminated.

Biological processes, e.g. growth of micro-organisms or chemical processes, e.g. oxidation, usually exchange gases with the atmosphere surrounding the process. For example oxidation process of a substance will withdraw oxygen from the atmosphere. A growing colony of micro-organisms may consume oxygen from the atmosphere and release gaseous substances like carbon dioxide or other substances depending on the specific micro-organism that is present and the available nutrient(s).
Various methods are available to measure a concentration of gaseous substances in an atmosphere. These methods measuring a parameter over time that is representative for a composition of said atmosphere,
One such method is a hot-plate based semiconductor gas sensor method. According to this method a metal oxide resistance that has a high sensitivity to particular gases is cyclically heated. The resistance is for example a SnO2 resistance, which is sensitive to carbon monoxide and methane. A sensor of this type carried out as a micro hot-plate is described for example in WO0250528 as well as in Barretino et al. "Hotplate-based conductometric monolithic CMOS gas sensor system", 2003 Symposium on VLSI circuits pp. 156-160.

Another method uses a polymer based sensor comprising a detector array made of carbon black particles blended with different polymers. The polymers show a mutually different expansion in response to the presence of gases. The carbon black particles form a conductive structure, the conductivity of which depends on the amount of expansion. Hence, the conductivity is indicative for the type and concentration of the gases to which the detectors are exposed. The operation thereof is described in detail in
http://archives.sensorsmag.com/articles/0800/56/main.shtml.
Now it has been observed by the inventor that such methods are very suitable for identifying a process wherein gaseous substances are exchanged with an atmosphere, when an observed function of the measured parameter is segmented in time windows, wherein boundaries of the time windows correspond to changes in behaviour of said at least one parameter. The resulting segmentation in time windows strongly depends on the type of the underlying process. Steps in the underlying process cause clearly visible changes in behaviors of the at least one observed parameter. Where such a step in the process to be investigated takes place, the function has relatively strong variations in comparison to neighboring portions. Accordingly, identifying such portions of the function gives valuable information about steps in the underlying process. Preferably, the identification comprises the step of comparing the function with at least one portion of the function observed for a known process.

The device may comprise a sensor as described above, e.g. a hot-plate sensor or a polymer based sensor, or another sensor for measuring at least one parameter over time that is representative for a composition of said atmosphere.
The device further comprises a signal processing facility for out the segmentation algorithm, for determining correspondence with steps of at least one predetermined process and for identifying the process. The signal processing facility may be carried out by dedicated hardware, by suitably programmed general processor or by a combination of both. For many applications the changes in the behavior of the observed parameter are at a relatively long time scale. E.g. whereas patterns to be recognized in audio signals have a time-scale in the order of milliseconds, the patterns in the observed parameter representative for the growth of a micro-organism is in the order of minutes or hours. Hence signal processing algorithms suitable for audio are also suitable for the present application, and can even be implemented with relatively low speed processors.

### BRIEF DESCRIPTION OF THE DRAWINGS

These and other aspects are described in more detail with reference to the drawing. Therein:
Figure 1 shows a first embodiment of a device according to the invention,
Figure 2A, 2B shows two further embodiments of a device according to the invention,
Figure 3 shows a part of a device according to the invention in more detail,
Figure 4 shows a response measured for a sensor in a device according to the invention,
Figure 5 shows a further response measured for a sensor in a device according to the invention,
Figure 6A, 6B respectively show a way of reducing data obtained from the response as shown in Figure 5,
Figure 7 shows a complete measurement of a response for a particular process,
Figure 8A-E shows characteristic portions in a response for various processes,
Figure 9 shows a first embodiment of a method according to the invention,
Figure 10A, 10B show a second and a third embodiment of a method according to the invention,
Figure 11 shows a parameter measured as a function of time for a micro-organism in response to a composition of nutrients,
Figure 12 shows a further embodiment of a device according to the invention.

### DETAILED DESCRIPTION OF EMBODIMENTS

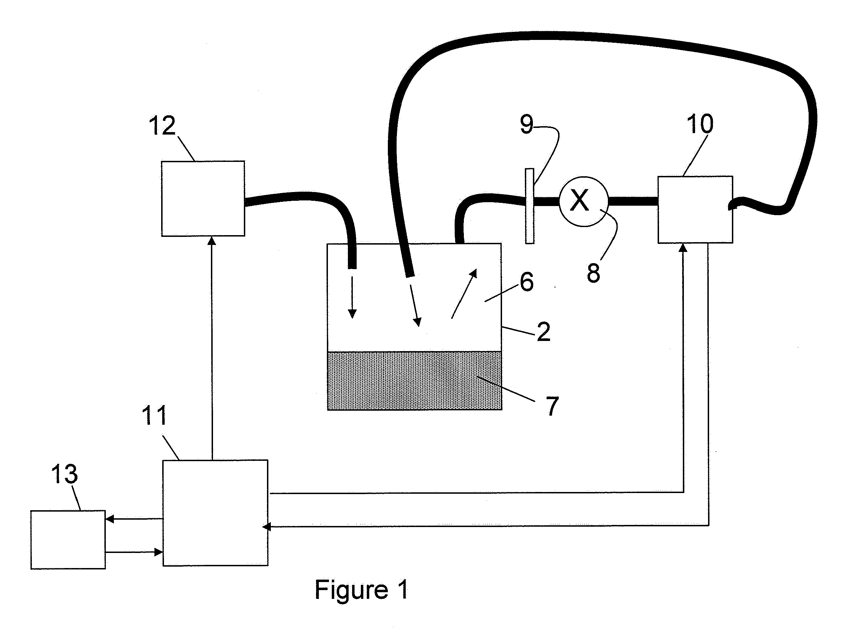

In the following detailed description numerous specific details are set forth in order to provide a thorough understanding of the present invention. However, it will be understood by one skilled in the art that the present invention may be practiced without these specific details. In other instances, well known methods, procedures, and components have not been described in detail so as not to obscure aspects of the present invention.
An embodiment of a device according to the invention is shown in Figure 1. In the embodiment of Figure 1 a closed system is provided, wherein a pump 8 draws the gas from the headspace 6 of a broth 7 in a vial 2 via a filter 9 over the sensor 10 and returns the headspace gas back to the headspace 6. The sensor 10 provides its measurement results to and is controlled by a micro-processor 11. The microprocessor 11 further controls a dosing device 12. The microprocessor 11 is coupled to a transmitter 13. The transmitter 13 may for example transmit the measurement results or a signal indicative for a status of the measurement to a base station. The transmitter 13 may be combined with a receiver, to enable a remote control of the device.

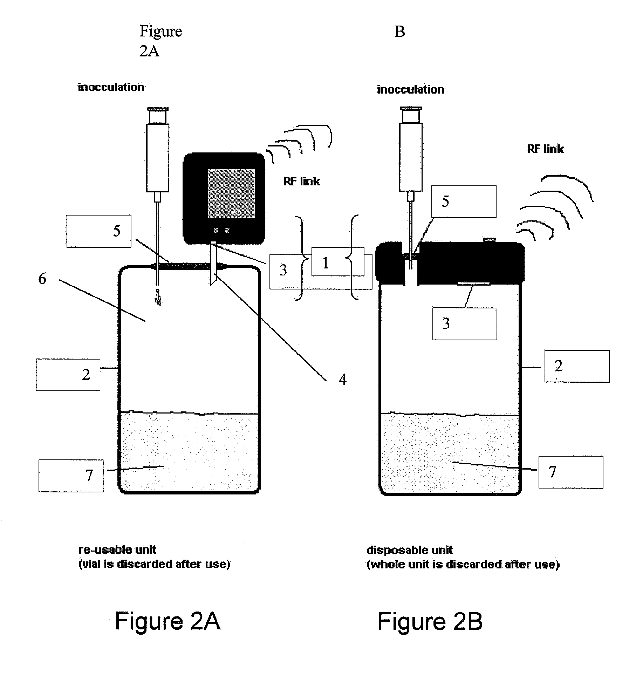

Figure 2A and 2B show two other exemplary embodiments of a device according to the present invention. Parts therein corresponding to those in Figure 1 have the same reference number. A vial is shown comprising a broth with a composition of nutrients and a micro-organism to be classified. The development of the micro-organisms is a process that exchanges gaseous substances with an atmosphere. A sensor is arranged for measuring at least one parameter over time that is representative for a composition of said atmosphere. The atmosphere is enclosed by the cap and or septum that covers the vial. Although it is not mandatory that the atmosphere is enclosed, this is favorable for the reliability of the measurement. In this embodiment, the sensor will measure the headspace 6 over the broth 7 provided in the vial 2 via simple diffusion without any mechanical pump.

In the embodiment of Figure 2B a special cap 1 is provided that can be placed on the fermentation bottle or vial 2. The cap 1 typically comprises the sensors, the signal processing device and may also comprise a communication (RF) device to transmit information to a remote processor. Alternatively the cap may for example comprise a connector for a wired connection of the signal processing device to the remote processor. In again another embodiment the cap may contain a memory for storing the measurements, to enable these measurements to be processed off-line.

The detector 3 in Figure 2A comprises a puncturing device 4 for puncturing a septum 5 provided on said vial 2. This detector 3 can be recycled by sterilizing and/or replacement of the puncturing device 4.

In Figure 2B, the cap 1 and the detector 3 are provided integral. In this embodiment, the cap 1 comprises a septum 5 to be punctured while inserting a micro-organism test substance through inoculation.

A detector unit according to the present invention as shown in Figures 1, 2A and 2B has distinct advantages over other detection systems such as pressure/optical CO2 measurement and colorizing reactions. The detection level of the e-nose is in the order of magnitude of several ppm, and the detector also makes distinction of the gas composition possible at a very early stage. In addition, in contrast to the present invention, colorizing reactions and monitoring of CO2/02 production/consumption require large amounts of reactants or fermentation times.

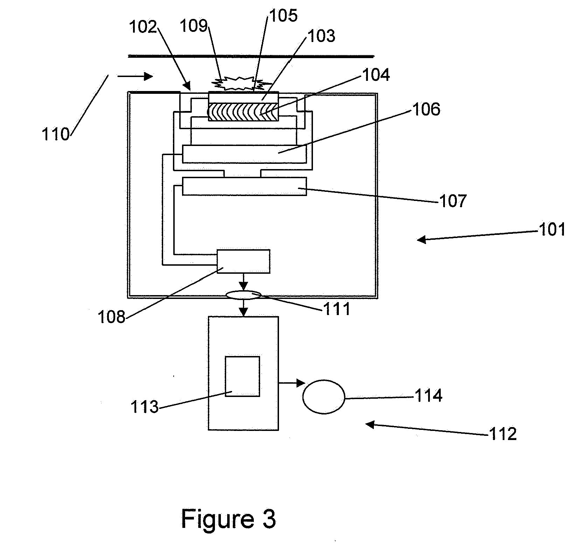

Turning to Figure 3 a typical layout is shown for a detection unit, in particular detector 101 implementing a heatable conducting plate 102, also known as hotplate sensor 102. The hotplate sensor 102 is typically provided by a metal oxide sensor element 103 which is sensitive to chemical reactions taking place near the sensor surface area, that is in close spatial relationship with a heater element 104. This sensor element 103 shows in particular a variation in conductance depending on chemical traces reacting near the exposed surface area 105 thereof. Various metal oxide sensor elements 103 are known, including but not limited to tin oxide, zinc oxide, iron oxide and tungsten oxide sensors with or without added catalyst, including but not limited to platinum and palladium.

The hotplate 102 is heated by a heater element 104 which is preferably attached in close vicinity of the sensor element 103 produced by MEMS (micro electrical mechanical systems) technology thus ensuring an identical temperature of the conducting sensor element 103 and the heater element 104. The heater element 104 has a low thermal mass and is controlled by a processor 106 for to provide a stabilized temperature in said sensor element 103. Typically this is provided by a balancing circuit implementing a Wheatstone bridge as is further elucidated in European Patent Application 07105509.9 filed earlier by the same Applicant.

Furthermore, the sensor element 103 is connected to a detection circuit 107 for detecting a change of resistance in the sensor element 103 in accordance with the presence of a chemical trace reacting in the presence of the conducting plate. The outputs of the detection circuit 107 in connection with a preset temperature provided by the processor 106 are stored in an internal memory element 108 of the detector, which can be any type of memory, typically a flash memory.

In the memory element 108, among others, a plurality of detected resistance values in the detection circuit relative to a plurality of preset temperatures can be stored to form a footprint of a number of chemical substances 109 which are sensed by the hotplate 102 by exposing the hotplate to a flow of gas 110. Alternatively, the hotplate can be subjected to stagnant air.

In the embodiment shown, the results are stored in the memory element 108 to be transmitted via a communication terminal 111 to a base station 112 comprising a database for storing footprints of predetermined chemical substances. Thus the stored footprints can be communicated to the base station 112 comprising a database 113, for providing a best match 114 of any of said stored footprints in the memory element 108 to any of footprints stored in the database 113 of known chemical substances. In this way a particular detected composition of chemical substances can be identified in the database 113 via per se known pattern recognition and identification software techniques.

Although in this embodiment, the identification of a sensed chemical composition can be done online or offline in an external base station 112, the detector 101 may also be equipped with specific matching routines which can match the detected footprint with one or more predefined chemical substances on board of the detector 101. In this way, the detector 101 can be easily modified to provide a detector for detecting specific predetermined chemical substances. In this (not shown) embodiment, the detector 101 hence comprises in addition a comparison circuit for comparing a stored footprint with a predetermined set of pre-stored footprints of predetermined chemical substances, so as to determine a particular detected chemical substance.

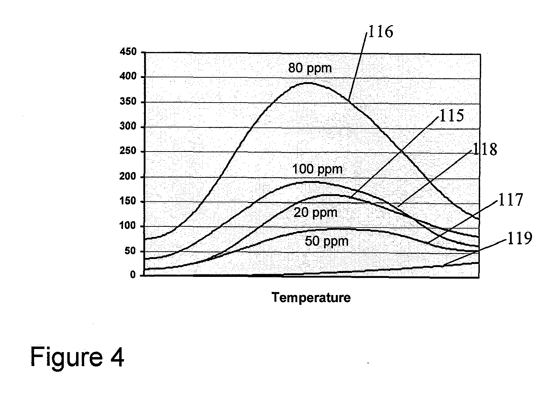

Figure 4 shows a response characteristic of a heatable metal oxide sensor that is exposed to a variety of compositions or chemical substances for a concentration of 20 and 80 ppm (curve 115 and 116 respectively) of toluene and for a concentration of 50 and 100 ppm (curve 117 and 118 respectively) of butyl acetate. Also a blank response 119 is shown, illustrating a detected conductance for varying temperatures. The typical detection temperatures vary between 200 and 600 °C. It can be shown generally that the metal oxide sensor produces peak conductance values for different chemical substances on different temperature values and for different peak values. For example, the conductance for toluene is generally higher than for butyl acetate.

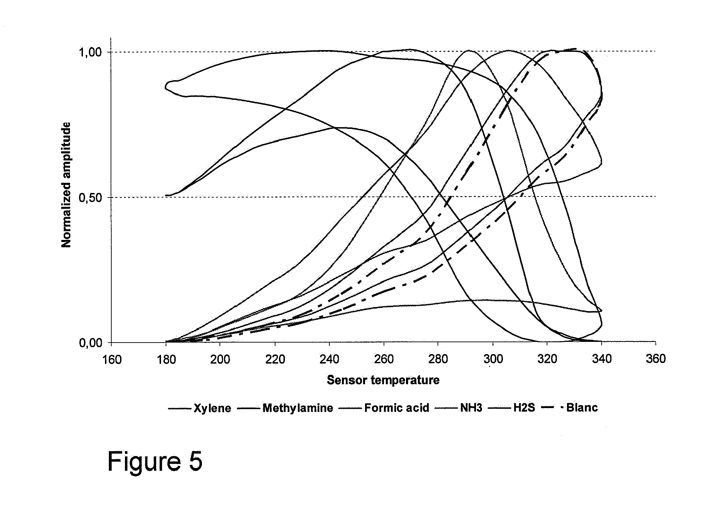

Figure 5 shows a detector response for a thermal cycle, that is, for a detector that is arranged to increase a sensing temperature of the detector, in particular, of the hotplate 102 from substantially 180 °C to substantially 340 °C while measuring a sensor response. To this end, a normalized amplitude is shown on the Y-axis of the graph; wherein a thermal cycle is passed by a temperature that varies substantially sinusoidal in time. This response is preferably fixed within a predetermined time frame, of substantially a few seconds, typically from about 5 to 20 seconds.

A normalized amplitude can be a good measure for a typical detector response to various gaseous substances; responses are shown for various substances, in particular, Xylene, Methylamine, Formic Acid, NH3 and H2S. For reference, a blank response is also shown.

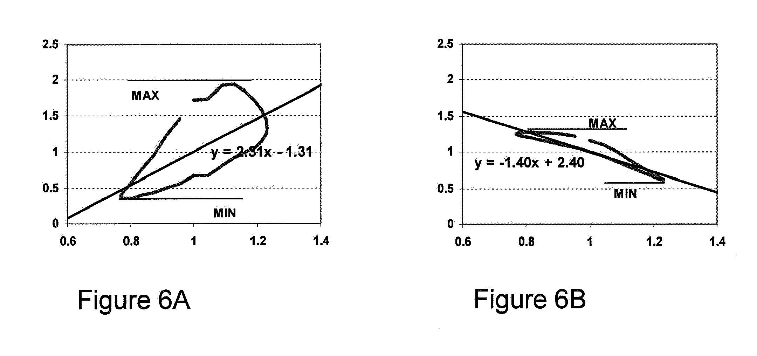

Figure 6 schematically shows how the data measured during a temperature cycle may be reduced into a minimum value MIN, a maximum value MAX and a slope α. The slope α is defined as the ration dy/dx, wherein y indicates the measured amplitude of the sensor and x is the temperature of the hot-plate.

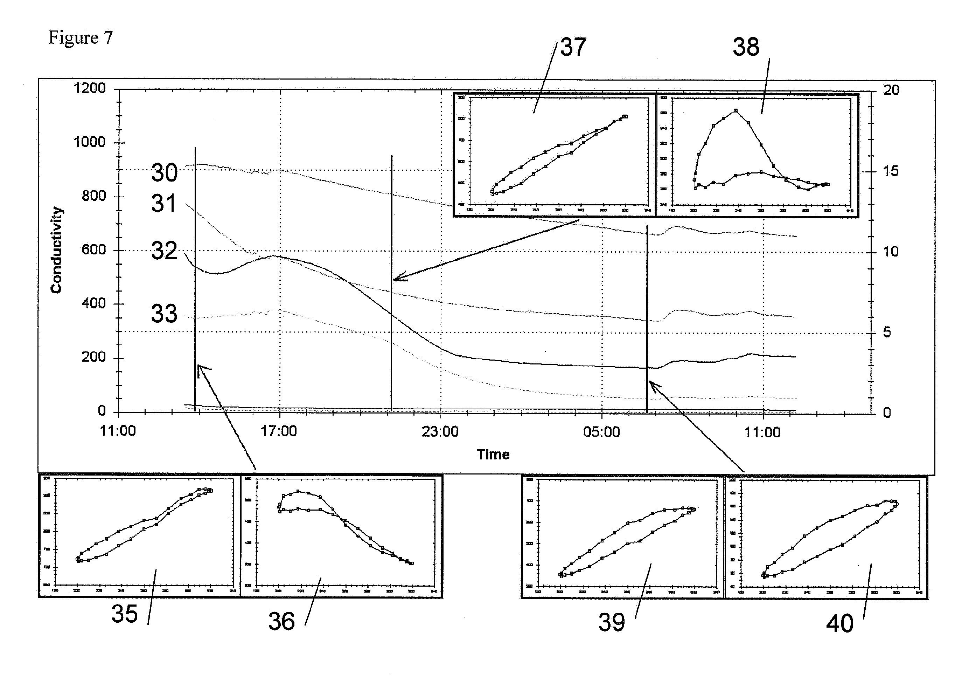

Figure 7 shows an illustrative graph of a growth test of a pseudomonas bacterial culture on a broth.

The test of Figure 7 was performed at a relatively low ambient temperature of 18 degrees. At optimal temperatures the growth times can be expected to shorten considerably, for example, so that a test can be completed within 3-4 hours.

Conductivity outputs of a palladium catalyzed SnO2 sensor (upper two lines 30 and 31) and a platinum catalyzed SnO2 sensor (lower two lines 32 and 33) were measured. The upper two lines 30, 31 represent measured maxima; minima respectively, measured in time during a temperature cycle of the palladium catalyzed SnO2 detector. The "snap-shots" 35 and 36 are showing normalized graphs of temperature cycles on a specific moment in time for platinum- and palladium catalyzed SnO2 sensors respectively; that is three hours after start. Likewise snap-shots 37 and 38; resp 39 and 40 are measured temperature cycles after 10 hours and after 20 hours respectively; for platinum and palladium catalyzed SnO2 sensors respectively.

Accordingly, over time a substance concentration in said vial indicative of the presence or absence of a predetermined micro-organism is detected through the conductivity measurement.

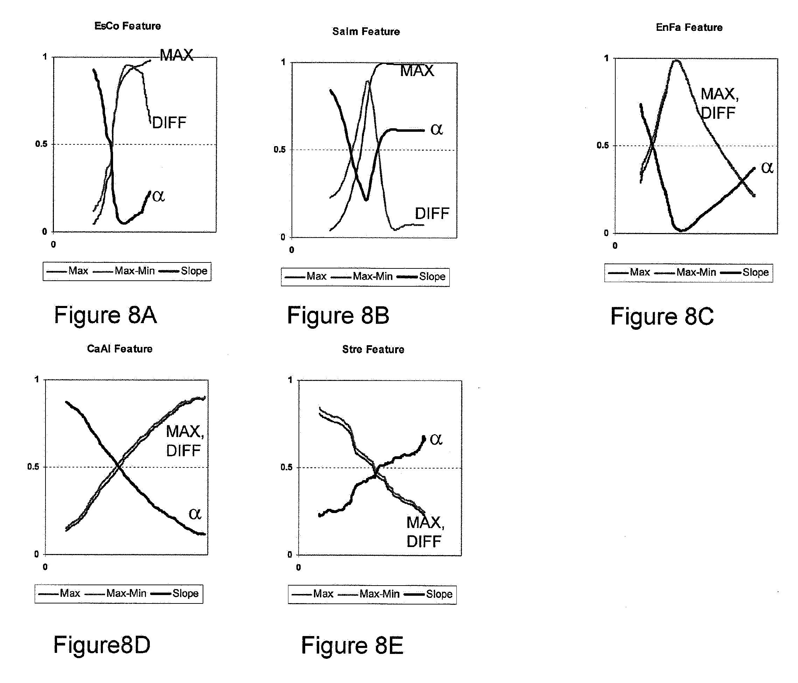

Accordingly, the method shows that a substance associated with a predetermined micro-organism or class of micro-organism can be tested that may be relevant to discriminate for clinical purposes. The substance can be a nutrient and/ or metabolite of said micro-organism. Typically the micro-organism is a bacterial or fungal pathogen, to be tested for in clinical purposes. For instance, as a nutrient, nitrogen, sulfur or carbon containing substances may be added. Depending on a measured (metabolic) response, a classification can be made of the micro-organisms to be tested.
For example certain yeasts will convert glucose to ethanol and/or methanol, Lactic acid bacteria will convert lactic sugar to lactic acid, certain anaerobic types are capable of generating ammonia or H2S (sulphate reducing bacteria). The continuous measurements of the detector enable continuous tracking of the headspace and thus enable following the metabolizing reactions over time. By careful selection of the composition of the broth, the monitoring over time allows following the sequential changes in metabolizing reactions/pathways as nutrients are depleted in preferred order. When a plurality of sparse nutrients is simultaneously available, the micro-organism will first select a first preferred type of the available nutrients. When this preferred nutrient is consumed the micro-organism selects a next available nutrient, until all nutrients are consumed. The selection of a next nutrient is detectable as a change in a behavior of a parameter indicative for the composition of the atmosphere associated with the broth. The micro-organism may for example consume more oxygen in order to metabolize the new nutrient, or may generate other gases. Each time the micro-organism selects a next nutrient to consume, a change occurs in the behavior of the observed parameter.
Examples of micro-organisms which may be classified in this way are shown in Table 3. Figures 8A-E shows the response measured for 5 different groups of micro-organisms upon a change from the first preferred nutrient to the one but preferred nutrient in the Todd-Hewitt medium. The parameters measured as response are the square root of the maximum conductivity, the square root of the difference between the maximum and the minimum conductivity and the slope measured during successive temperature cycles of the sensor.
Finally, the resulting three variables sqrt(maximum), sqrt(maximum-minimum) and slope(normalized thermal cycle) were scaled to the range [0,1] over the full timescale of the individual experiment. The resulting values are indicated in Fig. 8A-E respectively by MAX, DIFF and α. Generally, the relationship between the concentration of a gas and the sensor response resembles the square root relationship. Scaling in this way preserves the relative importance of the response at lower concentrations. Figure 8A-E show the resulting simplified values obtained using this pre-processing procedure measured during successive temperature cycles.
The investigated micro-organisms are respectively Escheria Coli, Salmonella, Enterobacter Faecalis, Candida Albicans, Streptococcus Haemolyticus,

As is demonstrated in Figure 8A-E different micro-organisms respond mutually substantially differently to the same broth. For example from Figure 8A it is clear for Escheria Coli that upon the change from the first preferred nutrient to the second preferred nutrient in the Todd-Hewitt medium the normalized difference (DIFF) rises from a value close to 0 to a peak value close to 1, and subsequently lowers to about 0.6. The normalized maximum conductivity (MAX) rises from a value close to 0 to a value close to 1 and keeps rising after the normalized difference (DIFF) lowers. The normalized slope (α) lowers from a value close to 1 to a value close to 0 and subsequently rises again to about 0.2. The behavior observed for Streptococcus Haemolyticus in Figure 8E is substantially different for example. Both the normalized maximum (MAX) and the normalized difference (DIFF) diminish from a value in the range of 0.8 to a value of about 0.3. The normalized slope (α) increases from a value of about 0.2 to a value of about 0.6. Accordingly the change of nutrients results in a clear signature of the tested micro-organism.
Various compositions of nutrients may be used. As an alternative to the Todd-Hewitt bouillon (Table 1) a composition known as the Mueller-Hinton bouillon (Table 2) may be used.
It is not essential to know which nutrients are selected by the micro-organism in what order. It is only essential that the change of nutrient results in a detectable and reproducible change in the observed function.
An advantage of providing the micro-organism with a mixture of nutrients, such as these bouillons is that the "choice" of the micro-organism, apart from its response is indicative for the kind of micro-organism. This could be valuable if two types of micro-organism have the same response to the same nutrients, but prefer to consume the nutrients in a different order.
Instead of offering the nutrients simultaneously and let the micro-organisms determine the order in which they are consumed, it is alternatively possible to provide the micro-organism subsequently with the mutually different nutrients and to determine the response of the micro-organism in response thereto. This is illustrated for example in the embodiment of Figure 1.

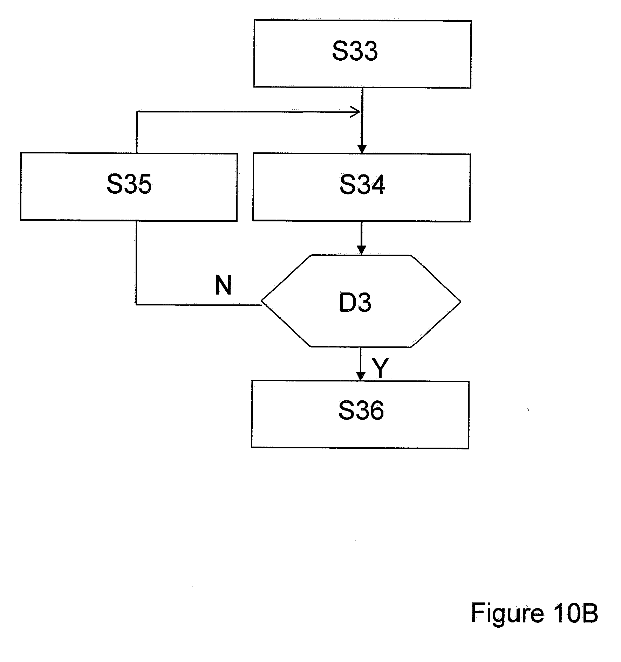

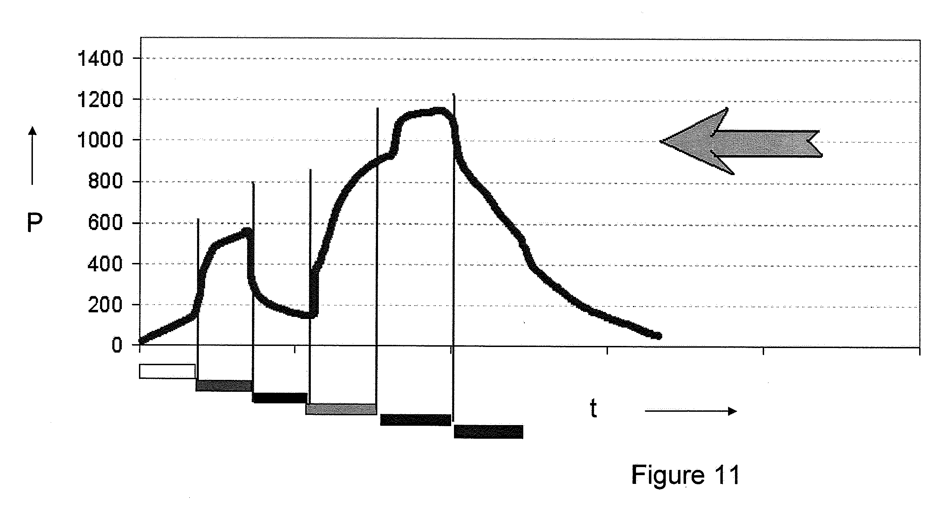

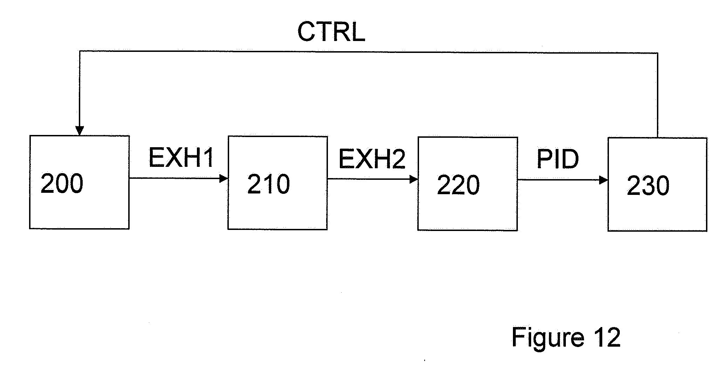

Figure 1 schematically shows a measurement arrangement in which the vial is provided with a dosing and selection arrangement 12. The dosing arrangement, controlled by micro-processor 11 can provide various nutrients to the broth at predetermined points in time.
This has the advantage that the order of the nutrients can be controlled as an independent parameter, and that it is known beforehand at which point in time the relatively strong variations in the observed function will occur.

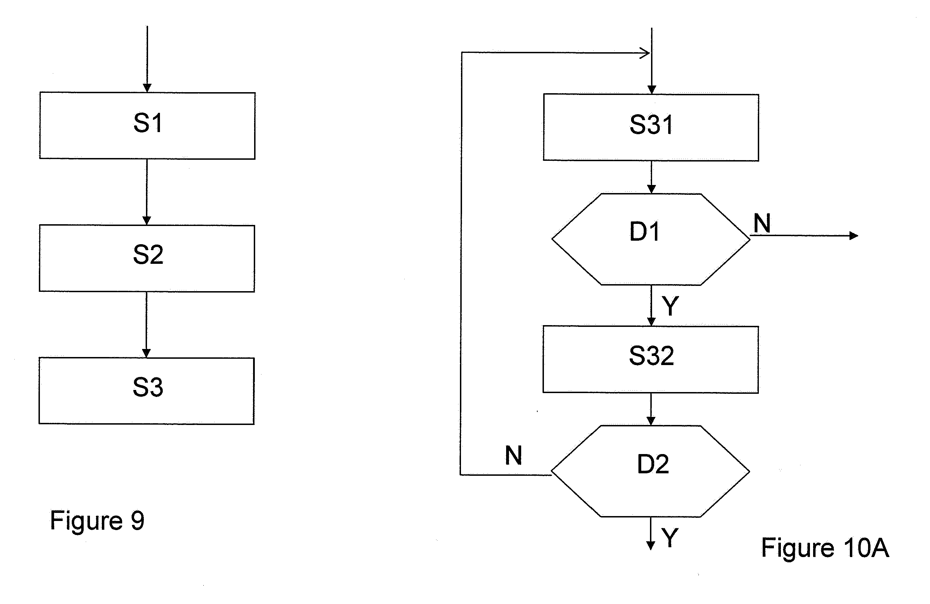

Figure 9 schematically shows a method according to the invention for identifying a process wherein gaseous substances are exchanged with an atmosphere.
In a first step S1 of the method at least one parameter is measured that is indicative for a composition of said atmosphere. The parameter is measured as a function of time.
In a second step S2 of the method at least one portion in said function is identified that has a relatively strong variation in comparison to neighboring portions,
In a third step S3 the process is identified on the basis of the identification of said at least one portion.
Under equal circumstances, i.e. the same temperature, the same nutrients in the same quantities, a micro-organism will show the same growth, and accordingly the same behavior for the selected parameter will be measured.
In these circumstances it is relatively simple to compare the measured function with the sample portions of a function measured for a known process. There may however be circumstances that this is not possible. For example the test may have to be carried out at another ambient temperature than for which the sample portions were measured. In that case a so called "time-warping" or "elastic matching" may be applied to map the observed behavior of the selected parameter to measurements for known substances. In other words a sequence of first values measured for said parameter at subsequent points in time is mapped to corresponding second values in a predetermined sequence of values for said parameter at subsequent points in time representative for the known process. Therein the order of the first values corresponds to the order of the second values, and the mapping allows first values having a first temporal distance to be mapped to second values having a second temporal distance, differing from the first temporal distance. Various algorithms, such as Dynamic Time Warping (DTW), longest common subsequence (LCSS), Time Warped Longest common subsequence (TW_LCSS), Minimal Variance Matching (MVM) are known from the field of data-mining, speech (sound) recognition and gesture analysis. A particular useful algorithm is the MVM algorithm
An algorithm known as Minimal Variance Matching (MVM) is described for example by L.J. Latecki, V. Megalooikonomou, Q.Wang, R. Lakaemper, C.A. Ratanamahatana, and E. Keogh, "Elastic Partial Matching of Time Series", in A. Jorge et al. (Eds.): PKDD 2005, LNAI 3721, pp. 577-584, 2005. This algorithm is specifically adapted for non-discrete values in time series. Generally speaking, a cost matrix is calculated and an optimal path is calculated. In this case the cost matrix is the matrix of Euclidian distances between points of both time series. The algorithm includes skipping capabilities that enable it to match sub-parts of time series.
For the purpose of matching features of processes related to growth of micro-organisms, it has to be taken into account that relatively short sub-features have to be recognized over a much longer full odor curve. These sub-features are the changes in the behavior of the measured parameter due to changes in the metabolition of the micro-organism that is investigated. The latter gives rise to matches in which the sub-feature can be spread over large portions of the full odor curve giving relatively low path costs, as the MVM algorithm allows skipping over parts of the full odor curve. This entails the risk of false matches. To counter this, an amended algorithm is proposed as shown in Figure 10A. This algorithm is based on the MVM algorithm discussed above. The amount of skipping is controlled however. Therein a maximum skip parameter is introduced. Once in decision D1 it is determined that the starting point of the sub-feature is found in a first part S31 of the algorithm, this 'max_skip' parameter is used to force subsequent points of the sub-feature to be matched in a second part S32 of the algorithm in such a way that a maximum of 'max_skip' points of the full odor curve may be skipped. If it is determined in D2 that a subsequent point of the sub-feature is matched in the second part S32, it is determined that the sub-feature is detected. If it is determined in D2 that the subsequent point could not be found than part S31 of the algorithm, is repeated to attempt to find a subsequent first point of a sub-feature.
This prevents the algorithm from finding a false match by skipping large parts of the full odor curve.
The introduction of the 'max-skip' parameter enforces a more rigid matching of sub-features. The max-skip parameter indicates the maximum number of points that may be skipped in the function to be matched for the function it is to be compared with. However, due to the variability of the obtained odor curves, often the starting point of the match may trigger on a non-optimal point early in the full odor curve and miss the actual feature later on in the full odor curve. This is solved by a second modification. Therein the MVM algorithm or another elastic-matching algorithm is repeatedly applied to subsequent windows within the measured functions. The windows typically have a size equal to the width of the samples for the known process times the max_skip parameter) in a window of data taken from the full odor curve. The window preferably has a size of the number of elements of the sub-feature multiplied by the max_skip parameter. The 'window' is slid over the full odor curve and the best possible match with all windows is obtained. This is illustrated in Figure 10B. In step S33 the first window is selected from the target function (the function measured for the unknown process). In step S34 the best match is determined for the sample of the known process with the portion of the target function in said window. In step D3 it is determined whether the last step S34 was carried out for the last window. If this is not the case the next window is selected in step S35. If step S34 was indeed carried out for all windows the best match found for each of the windows is selected in S36.
Subsequent to step S36 the process may be identified if the best match found complies with a predetermined norm. For example a distance measure may be used, and if the distance found for said match is less than a predetermined distance it is determined that the process is the known process for which the sample was obtained.
Alternatively the algorithm of Figure 11 may be repeated for a plurality of samples from mutually different known processes. And the process under investigation may be identified as the known process for which the sample gives the best match.
The combination of these two adaptations resulted in a 100% correct match of sub-features in all full odor curves of the group from which they were derived. In an experiment a set of five devices according to the invention, denoted with codes 43, 6D, F0, F3, FC were used to classify various micro-organisms. Table 2 shows an overview of the measurements carried out for these micro-organisms
Each device comprises an array of three semiconductor gas sensors, a palladium catalyzed SnO2 sensor, a platinum catalyzed SnO2 sensor and an SnO2 sensor without catalyst, which are thermally cycled. The response of the sensors is recorded as function of the working temperature. All three sensors complete a thermal cycle every 20 seconds. The palladium catalyzed SnO2 sensor was found to give the most discriminating results. The temperatures of the sensors are automatically calibrated, and differ less than 0.5% from unit to unit over the full working temperature range. The devices according to the invention are equipped with high quality low-flow membrane pumps that draw air and return it to an enclosed space. This system was used to monitor small volumes of headspaces over long periods of time.
Figure 1 schematically shows the example set-up. Membrane filters were used to prevent contamination of the devices and contamination of subsequent experiments.
The five sub-features as described with reference to Figure 8A-E, were matched against all odor growth curves. The resulting path costs were divided by the number of elements in the sub-feature and then multiplied by 100 for normalization. The results are given in table 5. Note that a score of 0 means a perfect match. The larger the score, the more imperfect the match is (the higher the cost is). In essence, the matching of a sub-feature is the likelihood of the occurrence of specific metabolizing behavior in time.
The top half of table 5 (above the dividing line) contains the scores of the odor curves from which the sub-features were extracted. The bottom half are the scores of the odor curves which were not used for sub-feature extraction, as on visual inspection these curves did not exhibit any feature which was not apparent in the first group.
The features for which minimum path costs were found are indicated in bold. From these measurements it was concluded that the five types of micro-organisms could be classified with an accuracy in the range of 67% for Candida Albicans upto an accuracy of 100% for Salmonella.
The accuracy of the identification of the process may be further improved if the micro-organism is provided with a relatively large plurality of sparse nutrients.
By way of example this is shown in Figure 11 for a composition with 6 nutrients. The nutrients may be dosed in an amount that forces the micro-organism to step to a new nutrient every few minutes (e.g. 5 to 40 minutes). At each change of nutrient a rapid variation is visible in the observed function of the measured parameter P as a function of time t, which can easily be discriminated from neighboring portions of the functions.
The invention is also applicable to chemical processes. By way of example this is shown in Figure 12 for a combustion engine 200 that exhausts exhaust gases EXH1 to catalyst 210. Both the operation of the engine 200 and that of the catalyst 210 depend on their operation temperature. The modified exhaust gases EXH2 are monitored by a detector 220 according to the invention that provides process identification information to a processor 230. The processor 230 on its turn provides control signals CTRL to the engine 200 for example to control a fuel/air composition or an ignition time.
Many variations will be apparent to the skilled person within the scope as defined by the claims. For example, instead of identifying the process by comparison with sample portions of functions observed for known processes, it is possible to derive characteristic features from the observed function. For example portions of the observed function may be identified wherein the function has a predetermined sequence of curvatures. Alternatively, a plurality of parameters may be measured, and it may be determined at which point in time the parameters have a predetermined relation.
In the claims the word "comprising" does not exclude other elements or steps, and the indefinite article "a" or "an" does not exclude a plurality. A single component or other unit may fulfill the functions of several items recited in the claims. The mere fact that certain measures are recited in mutually different claims does not indicate that a combination of these measures cannot be used to advantage. Any reference signs in the claims should not be construed as limiting the scope.

**Table 1. Composition of the Todd-Hewitt bouillon**

| Todd-Hewitt bouillon | |
|---|---|
| Component | Amount (g/l) |
| Heart infusion | 3,1 |
| Peptone | 20,0 |
| Glucose | 2,0 |
| Sodium carbonate | 2,5 |
| Sodium chloride | 2,0 |
| Disodium phosphate | 0,4 |

**Table 2. Composition of the Mueller-Hinton bouillon**

| Mueller Hinton bouillon | |
|---|---|
| Component | Amount (g/l) |
| Beef infusion | 2,0 |
| Casein peptone (acidic hydrolysate) | 17,5 |
| Corn starch | 1,5 |

**Table 3. Overview of micro-organisms**

| **Abbr.** | **Name** |
|---|---|
| CaAl | Candida Albicans |
| EnCl | Enterobacter Cloacae |
| EnFa | Enterobacter Faecalis |
| EnXX | Enterobacter XX |
| EsCo | Escherichia Coli |
| KlOx | Klebsiella Oxytoca |
| PrMi | Proteus Miribilis, |
| PsAu | Pseudomonas Aeruginosa |
| SaEn | Salmonella En., |
| SaSD | Salmonella SD., |
| SaTy | Salmonella Ty. |
| StAu | Staphylococcus Aureus |
| StEp | Staphylococcus Epidermis, |
| | Streptococcus |
| StHa | Haemolyticus. |

**Table 4. Overview of tests carried out.**

| Unit | Bact. | Inocc. | Date | Unit | Bact. | Inocc. | Date |
|---|---|---|---|---|---|---|---|
| 43 | EsCo | 1 | 11-06-07 | 43 | StHa | 1 | 16-07-07 |
| 43 | EsCo | 1 | 12-06-07 | 6D | StHa | 4 | 17-07-07 |
| 43 | EsCo | 2 | 10-07-07 | F0 | StHa | 3 | 16-07-07 |
| F0 | EsCo | 4 | 10-07-07 | F3 | StHa | 2 | 16-07-07 |
| F3 | EsCo | 2 | 11-06-07 | | | | |
| F3 | EsCo | 2 | 12-06-07 | 6D | KlOx | 1 | 11-07-07 |
| | | | | | | | |
| F3 | EsCo | 3 | 10-07-07 | FC | KlOx | 2 | 11-07-07 |
| 43 | SaEn | 1 | 17-07-07 | 6D | PrMi | 4 | 12-07-07 |
| F0 | SaEn | 1 | 18-07-07 | F3 | PrMi | 2 | 11-07-07 |
| 43 | SaSD | 1 | 18-07-07 | | | | |
| F3 | SaSD | 1 | 17-07-07 | 6D | EnCl | 2 | 10-07-07 |
| F3 | SaTy | 1 | 18-07-07 | F0 | EnCl | 1 | 9-07-07 |
| | | | | | | | |
| 43 | EnFa | 3 | 13-07-07 | 43 | EnXX | 1 | 19-07-07 |
| 6D | EnFa | 1 | 26-06-07 | F0 | EnXX | 2 | 19-07-07 |
| 6D | EnFa | 1 | 13-07-07 | F3 | EnXX | 2 | 19-07-07 |
| F3 | EnFa | 4 | 26-06-07 | | | | |
| F3 | EnFa | 4 | 13-07-07 | 43 | StAu | 4 | 14-07-07 |
| FC | EnFa | 2 | 26-06-07 | 6D | StAu | 1 | 11-06-07 |
| FC | EnFa | 2 | 13-07-07 | 6D | StAu | 1 | 12-06-07 |
| | | | | 6D | StAu | 2 | 14-07-07 |
| 43 | CaAl | 1 | 23-06-07 | F0 | StAu | 1 | 13-07-07 |
| 43 | CaAl | 1 | 25-06-07 | FC | StAu | 2 | 11-06-07 |
| 43 | CaAl | 3 | 9-07-07 | FC | StAu | 2 | 12-06-07 |
| 6D | CaAl | 1 | 9-07-07 | FC | StAu | 3 | 14-07-07 |
| F3 | CaAl | 4 | 9-07-07 | | | | |
| FC | CaAl | 2 | 9-07-07 | 43 | PsAe | 2 | 12-07-07 |
| | | | | 6D | PsAe | 2 | 23-06-07 |
| 6D | StEp | 3 | 16-07-07 | 6D | PsAe | 2 | 25-06-07 |
| F0 | StEp | 2 | 14-07-07 | F0 | PsAe | 4 | 12-07-07 |
| F3 | StEp | 1 | 14-07-07 | F3 | PsAe | 3 | 12-07-07 |
| FC | StEp | 4 | 16-07-07 | FC | PsAe | 1 | 23-06-07 |
| | | | | FC | PsAe | 1 | 25-06-07 |
| | | | | FC | PsAe | 1 | 12-07-07 |

**Table 5 Final results**

| | **F_EsCo** | **F_Salm** | **F_EnFa** | **F_CaAl** | **F_Stre** |
|---|---|---|---|---|---|
| 43_EsCo_1_T_0611 | **8.8** | 54.4 | 28.3 | 28.7 | 57.6 |
| 43_EsCo_1_T_0612 | **10.4** | 59.2 | 36.8 | 38.5 | 57.8 |
| 43_EsCo_2_T_0710 | **11.5** | 59.8 | 52.7 | 54.1 | 62.5 |
| F0_EsCo_4_T_0710 | 27.2 | 11.7 | 20.9 | 44.0 | 25.9 |
| F3_EsCo_2_T_0611 | **17.8** | 53.2 | 29.4 | 32.1 | 60.7 |
| F3_EsCo_2_T_0612 | **6.0** | 48.6 | 35.3 | 39.3 | 52.6 |
| F3_EsCo_3_T_0710 | **6.4** | 46.5 | 39.5 | 36.1 | 41.6 |
| 43_SaEn_1_T_0717 | 40.7 | **21.8** | 39.1 | 43.7 | 64.4 |
| 43_SaSD_1_T_0718 | 59.7 | **25.7** | 93.8 | 69.7 | 70.9 |
| F0_SaEn_1_T_0718 | 48.3 | **7.2** | 39.4 | 36.1 | 62.9 |
| F3_SaSD_1_T_0717 | 44.1 | **7.1** | 74.4 | 61.9 | 67.4 |
| F3_SaTy_1_T_0718 | 72.4 | **37.9** | 86.6 | 66.5 | 54.9 |
| 43_EnFa_3_T_0713 | 31.3 | 52.9 | **3.5** | 5.0 | 20.0 |
| 6D_EnFa_1_T_0626 | 32.3 | 54.4 | **3.9** | 8.8 | 24.4 |
| 6D_EnFa_1_T_0713 | 21.4 | 56.9 | **9.0** | 17.6 | 31.9 |
| F3_EnFa_4_T_0626 | 14.7 | 57.6 | **12.9** | 32.5 | 20.6 |
| F3_EnFa_4_T_0713 | 29.3 | 55.1 | **5.6** | 12.0 | 33.6 |
| FC_EnFa_2_T_0626 | 29.7 | 50.9 | **5.3** | 5.5 | 37.1 |
| FC_EnFa_2_T_0713 | 31.4 | 54.1 | **4.6** | 11.3 | 22.1 |
| 43_CaAl_1_T_0623 | 45.0 | 53.1 | 35.5 | **6.4** | 48.2 |
| 43_CaAl_1_T_0625 | 39.4 | 51.4 | 31.2 | **1.9** | 42.2 |
| 43_CaAl_3_T_0709 | 40.4 | 52.7 | 30.3 | **10.3** | 40.6 |
| 6D_CaAl_1_T_0709 | 23.8 | 54.1 | 22.3 | **12.5** | 38.7 |
| F3_CaAl_4_T_0709 | 24.3 | 55.2 | **6.2** | **11.2** | 18.7 |
| FC_CaAl_2_T_0709 | 37.4 | 48.6 | 36.1 | **10.5** | 8.7 |
| 43_StHa_1_T_0716 | 53.3 | 54.6 | 41.8 | 49.1 | **9.6** |
| 6D_StEp_3_T_0716 | 44.3 | 62.3 | 29.3 | 53.9 | **18.6** |
| 6D_StHa_4_T_0717 | 26.2 | 52.0 | 11.8 | 14.3 | 23.6 |
| F0_StEp_2_T_0714 | 38.5 | 57.1 | 26.0 | 26.2 | **10.8** |
| F0_StHa_3_T_0716 | 55.5 | 56.0 | 43.2 | 56.0 | **11.0** |
| F3_StEp_1_T_0714 | 46.7 | 53.0 | 31.5 | 31.3 | **10.2** |
| F3_StHa_2_T_0716 | 52.4 | 55.0 | 39.3 | 47.8 | **18.9** |
| FC_StEp_4_T_0716 | 59.0 | 56.2 | 43.4 | 58.4 | **4.6** |
| 6D_KlOx_1_T_0711 | **15.1** | 21.6 | 44.3 | 43.3 | 56.1 |
| FC_KlOx_2_T_0711 | **12.9** | 47.4 | 37.1 | 19.9 | 53.2 |
| 6D_PrMi_4_T_0712 | **31.8** | 38.4 | 68.2 | 61.3 | 76.6 |
| F3_PrMi_2_T_0711 | 39.0 | **32.1** | 53.9 | 62.8 | 66.9 |
| 6D_EnCl_2_T_0710 | **12.6** | 36.8 | 23.7 | 37.8 | 35.2 |
| F0_EnCl_1_T_0709 | 36.1 | **23.1** | 23.8 | 44.6 | 35.8 |
| 43_EnXX_1_T_0719 | 36.8 | 56.0 | **26.7** | 40.5 | 35.6 |
| F0_EnXX_2_T_0719 | 38.5 | 25.9 | *35.3* | 39.9 | **24.0** |
| F3_EnXX_2_T_0719 | 32.7 | 30.2 | **24.8** | 45.6 | 31.9 |
| 43_StAu_4_T_0714 | 46.0 | 49.7 | 36.6 | 25.3 | **25.3** |
| 6D_StAu_1_T_0611 | 64.1 | 54.8 | 50.1 | 61.3 | **4.1** |
| 6D_StAu_1_T_0612 | 37.6 | 49.5 | 22.6 | 24.0 | **9.6** |
| 6D_StAu_2_T_0714 | 45.1 | 52.1 | 40.2 | 26.9 | **14.9** |
| F0_StAu_1_T_0713 | 43.3 | 54.4 | 35.0 | **23.3** | *30.8* |
| FC_StAu_2_T_0611 | 48.8 | 55.5 | 35.2 | 55.3 | **6.1** |
| FC_StAu_2_T_0612 | 55.2 | 55.7 | 41.9 | 49.7 | **10.3** |
| FC_StAu_3_T_0714 | 46.4 | 52.1 | 34.8 | **23.3** | *37.3* |
| 43_PsAe_2_T_0712 | 63.3 | 63.3 | 65.3 | **47.4** | *48.0* |
| 6D_PsAe_2_T_0623 | 67.0 | 58.0 | 61.0 | 56.0 | **26.7** |
| 6D_PsAe_2_T_0625 | 49.0 | 61.6 | 36.3 | 53.3 | **24.7** |
| F0_PsAe_4_T_0712 | 51.8 | 57.7 | 40.1 | 50.8 | **15.9** |
| F3_PsAe_3_T_0712 | 43.5 | 61.5 | 45.5 | **38.7** | 56.3 |
| FC_PsAe_1_T_0623 | 70.8 | 54.3 | 58.9 | 59.1 | 11.5 |
| FC_PsAe_1_T_0625 | 79.7 | 59.8 | 67.7 | 66.7 | **8.2** |
| FC_PsAe_1_T_0712 | 59.8 | 53.5 | 46.6 | 55.1 | **7.9** |

## Claims

1. Method for identifying a process wherein gaseous substances are exchanged with an atmosphere comprising the steps of
- measuring as a function of time at least one parameter indicative for a composition of said atmosphere,
- identifying at least one portion in said function having a relatively strong variation in comparison to neighboring portions, said at least one portion corresponding to a change in the process,
- identifying the process on the basis of the identification of said at least one portion.

2. Method according to claim 1, wherein the process is the growth of a micro-organism and the identification of the process is used to identify the micro-organism.

3. Method according to claim 2, wherein the atmosphere is the atmosphere in a container wherein a sample of the micro-organism to be identified is provided with a plurality of mutually different nutrients and wherein the stages of the process are the metabolizing of the micro-organism of the different nutrients.

4. Method according to claim 3, wherein the micro-organism is subsequently provided with the mutually different nutrients.

5. Method according to claim 3, wherein the micro-organism is simultaneously provided with a plurality of sparse nutrients.

6. Method according to claim 1, wherein the step of classifying the process is followed by the step of transmitting an alert signal.

7. Method according to claim 6, wherein the alert signal is transmitted wirelessly.

8. Method according to claim 1, wherein the step of determining whether these changes correspond to stages in at least one process to be identified comprises the step of mapping a sequence of first values measured for said parameter at subsequent points in time to corresponding second values in a predetermined sequence of values for said parameter at subsequent points in time representative for a known process, wherein the order of the first values corresponds to the order of the second values, wherein said mapping allows first values having a first temporal distance to be mapped to second values having a second temporal distance, differing from the first temporal distance.

9. Method according to claim 8, comprising a first mapping stage wherein a difference between the first and a second temporal distance is limited to a first limit, and a second mapping stage wherein the difference between the first and a second temporal distance is limited to a second limit, less than the first limit, the second mapping stage being entered after a preliminary match is made in the first mapping stage.

10. Method according to claim 8, wherein the predetermined sequence is relatively short in comparison to the sequence of first values, and a mapping is repeated for subsequent portions of the sequence of first values, wherein a best match between the predetermined sequence and the subsequent portions of the sequence of first values is selected for classification.

11. Method according to claim 1, wherein a measurement setting is periodically varied and for each period at least one of the following functions of the measured parameter is determined, an extreme value for said parameter, a difference between the highest value and the lowest value of said parameter and a correlation between said parameter and said setting.

12. Method for detecting according to claim 11, wherein the setting is a temperature of a hot-plate sensor.

13. Method for detecting according to claim 12, wherein the parameter is a resistance of the hot-plate sensor.

14. Device for identifying a process wherein gaseous substances are exchanged with an atmosphere (6), comprising
- a sensor (11) for measuring as a function of time at least one parameter that is representative for a composition of said atmosphere (6),
- a signal processing facility (11) for
- identifying at least one portion in said function having a relatively strong variation in comparison to neighbouring portions, said at least one portion corresponding to a change in the process,
- identifying the process on the basis of the identification of said at least one portion.

15. Device according to claim 14, wherein the process is the growth of a micro-organism and the identification of the process is used to identify the micro-organism, the device further comprising a facility (12) to subsequently provide the micro-organism with mutually different nutrients.

16. Device according to claim 1, wherein the signal processing facility is further arranged to determine whether the segments of the observed function correspond to stages in at least one process to be identified by mapping a sequence of first values measured for said parameter at subsequent points in time to corresponding second values in a predetermined sequence of values for said parameter at subsequent points in time representative for a known process, wherein the order of the first values corresponds to the order of the second values, wherein said mapping allows first values having a first temporal distance to be mapped to second values having a second temporal distance, differing from the first temporal distance.

17. Device according to claim 16, wherein said signal processing facility is arranged to execute a first mapping stage wherein a difference between the first and the second temporal distance is limited to a first limit, and to execute a second mapping stage after a preliminary match is found in the first mapping stage, in which second mapping stage the difference between the first and the second temporal distance is limited to a second value, less than the first limit.

18. Device according to claim 16 or 17, wherein the predetermined sequence is relatively short in comparison to the sequence of measured values, and the processing facility repeats a mapping for subsequent portions of the sequence of first values, wherein a best match between the predetermined sequence and the subsequent portions of the sequence of first values is selected for classification.

19. Device according to claim 14, wherein a measurement setting is periodically varied and for each period at least one of the following functions of the measured parameter is determined, an extreme value for said parameter, a difference between the highest value and the lowest value of said parameter and a correlation between said parameter and said setting.

20. Device according to claim 19, wherein the sensor is a hot-plate sensor (102) and the setting is a temperature of the hot-plate sensor.

21. Device according to claim 20, wherein the parameter is a resistance of the hot-plate sensor.

22. Device according to claim 14, further comprising a signaling facility for signaling that the step of identifying is completed.

23. Device according to claim 22, wherein the signaling facility (13) is a wireless facility.
